# EUROPEAN PATENT APPLICATION

(11) **EP 0 570 591 A1**
(43) Date of publication of application: **24.11.1993**
(21) Application number: 92924876.3
(22) Date of filing: 03.12.1992
(51) Int. Cl.: A23L 1/22, C07K 3/02, C07K 15/14

(54) **PROCESS FOR PRODUCING SWEETNESS INDUCER MIRACULIN**

(30) Priority: 04.12.1991 JP 347734/91
(71) Applicant: MITSUBISHI OIL CO., LTD., Minato-ku, Tokyo (JP)
(72) Inventor: KAMIMURA, Naohisa, Kawaguchi-shi Saitama (JP); MAKINO, Tomoyuki c/o MITSUBISHI OIL CO., LTD., Kawasaki-shi Kanagawa 210 (JP); HIRANO, Akiko, Toyonaka-shi Osaka 561 (JP); KURIHARA, Yoshie, Setagaya-ku Tokyo 158 (JP)
(74) Representative: Hansen, Bernd, Dr. Dipl.-Chem.
(86) International application number: JP9201584
(87) International publication number: WO9310676

(57) **Abstract**

A process for producing a sweetness inducer miraculin by extracting from a miraculin-containing raw material with the use of an aqueous acidic buffer solution, which enables even such a pure miraculin as to have a stabilized sweetness inducer activity and being free from colored impurities to be efficiently produced on an industrial scale.

## Description

### TECHNICAL FIELD

This invention relates to a process for efficiently and stably producing the sweetness-inducing substance miraculin from a miraculin-containing material.

### BACKGROUND ART

*Richadella dulcifica*, which is a plant native to Western Africa and commonly called miracle fruit, has a distinguishing character that when a sour food is tasted immediately after the miracle fruit has been tasted, one can feel a sweet taste. It is known that this function is due to the sweetness-inducing substance miraculin.

Although the detailed mechanism of its function of inducing sweetness has never been clarified so far, the substance miraculin per se has been purified and thus is already available as a pure product which is a glycoprotein of a molecular weight of approximately 40,000 and its amino acid sequence has been already clarified (refer to JP-A-3-218400, the term "JP-A" as used herein means an "unexamined published Japanese patent application").

In order to produce this sweetness-inducing substance miraculin, the following methods have been proposed so far.
(1) In a method proposed by K. Kurihara and L.M. Beidler [Science, 161, 1241 (1968)], freeze-dried miracle fruit sarcocarp is homogenized in a carbonate buffer solution of pH 10.5 and thus an active portion is extracted. In accordance with a report of Y. Kurihara and S. Terasaki [Biochem. Biophys. Acta, 719, 444 (1982)], an extract obtained by the same extraction treatment is salted out by adding ammonium sulfate and the precipitate thus formed is dissolved in a phosphate buffer solution. Then the ammonium sulfate is removed by dialyzing and the obtained solution is further purified by, for example, isoelectric focusing. Thus miraculin is obtained.
(2) In a method described in JP-A-62-242700, miraculin is extracted from miracle fruit sarcocarp with the use of a neutral salt solution of about pH 6 to 9 as the extraction solvent.
(3) In a method described in JP-A-63-185349, miracle fruit sarcocarp is powdered and, after adding water, homogenized. Then the mixture is washed with water until the washing liquor is not colored any more. Next, a neutral aqueous solution of sodium chloride is added to the washed sarcocarp and thus miraculin is extracted. Then the extract is salted out with ammonium sulfate to thereby give a miraculin fraction as a precipitate. This precipitate is dissolved in a neutral phosphate buffer solution and purified by CM Sepharose ion exchange chromatography and affinity chromatography.

However, the extracts obtained by the above-mentioned methods (1) and (2) are contaminated with impurities, and thus are deeply colored, which is undesirable from the viewpoint of hue. Further, these coloring matters can hardly be eliminated, though various biochemical treatments including ammonium sulfate precipitation, ion exchange chromatography, affinity chromatography and isoelectric focusing have been unsuccessfully attempted.

Furthermore, there is a serious problem in that the impurities causing coloration are strongly bound to miraculin, and thus damage some of its activity. This not only makes it impossible to obtain highly purified miraculin, but also results in a decrease in the sweetness-inducing activity thereof.

To solve this problem, in the above-mentioned method (3), miracle fruit sarcocarp is washed until the washing liquor is not colored any more. In this method, however, water-washing should be repeated as a pre-extraction treatment until the washing liquor becomes colorless. Thus a considerably long time and much labor are required for the washing and separation. Furthermore, there is a risk that the prolonged pretreatment might exert some undesirable effects on miraculin, since the miraculin contained in the miracle fruit sarcocarp is less stable.

In addition, a miraculin-containing solution is liable to undergo the inactivation of the miraculin component, when the pH value is 2 or less or 11 or above. It is, therefore, undesirable to extract miraculin under excessively acidic or alkaline conditions. When the pretreatment of washing with water is omitted in the above method (3), the pH value of the extract is excessively lowered by various acidic substances contained in the impurities. As a result, there arises another problem of the inactivation of the miraculin component. In the extracting methods employed in recent years, miraculin is extracted with a neutral NaCl solution after washing with water as a pretreatment to thereby eliminate these impurities, since these impurities are seemingly soluble in water.

As described above, conventional methods for extracting miraculin suffer from various problems, namely, coloration and a decrease in the activity due to complexes wherein impurities are strongly bound to miraculin, a decrease in the activity due to various acidic substances which excessively lower the pH value, or the necessity of a long time for water-washing as a pretreatment in order to eliminate the impurities forming complexes with miraculin so as to prevent these phenomena. Thus, a process for more efficiently producing miraculin which is not colored and highly stable and not suffers from any decrease in activity is desired. In addition, there has been strongly required in the art to develop a process for industrially producing a large amount of miraculin, which can be formulated into a highly pure and active powder, from the viewpoint of the application of miraculin to foods, drinks and medicines as well as the viewpoints of storage and transportation thereof over a long period of time.

### DISCLOSURE OF THE INVENTION

Under these circumstances, the present inventors have paid particular attention to impurities, which are liable to form rigid complexes together with miraculin contained in miracle fruit during the extraction process, and have conducted extensive studies in order to efficiently produce clean miraculin of a stable activity without needing any pretreatment such as water-washing. As a result, the inventors have successfully found out that when miraculin is extracted by using an acidic buffer salt aqueous solution prepared by adding NaCl to an aqueous buffer solution of a pH value within a range of from 3.5 to 5.5, any pre-extraction treatment such as water-washing for eliminating impurities contained in miracle fruit becomes unnecessary and the excessive decrease in pH due to various acidic substances contained in the sarcocarp can be prevented and thus miraculin, which suffers from no inactivation of the miraculin activity and is scarcely colored, can be extracted. The present inventors have further found out that clean and highly stable miraculin can be thus obtained. The present invention has been completed on the basis of these findings.

More particularly, a fresh miracle fruit sarcocarp is frozen by using, for example, liquid N₂ and dried under reduced pressure of 10 to 100 mmHg. The freeze-dried sarcocarp thus obtained is powdered and an acidic buffer salt aqueous solution, which has been prepared by adding NaCl to an aqueous buffer solution of a pH value within a range of from 3.5 to 5.5 having a concentration of 0.5 M or less in such a manner as to give an NaCl concentration of 0.4 to 0.5 M, is added thereto at a ratio of from 5 to 50 ml per gram of the freeze-dried miracle fruit sarcocarp at a temperature ranging from 0 to 5°C. Then the resulting mixture is homogenized by, for example, an ultrasonic treatment. After centrifuging, the supernatant is collected and referred to as an extract. It is preferable to repeat this extraction treatment twice or more. As the acidic buffer salt aqueous solution to be used in the extraction treatment, those which are prepared by adding NaCl to a 0.1 to 0.2 M aqueous buffer solution capable of maintaining the pH value within a range of from 3.5 to 5.5 in such a manner as to give an NaCl concentration of from 0.4 to 0.5 M are preferable. It is still preferable to use those which are prepared by adding NaCl to an aqueous buffer solution of 0.1 M in such a manner as to give an NaCl concentration of 0.5 M, though the acidic buffer salt aqueous solution is not particularly limited so long as it satisfies the above-mentioned requirements. For example, a monosodium citrate/disodium citrate solution (0.1 M), an acetic acid/sodium acetate solution (0.1 M) or a monopotassium phthalate solution (0.1 M) is used and, after adjusting the pH value, NaCl is added in such a manner as to give an NaCl concentration of 0.5 M.

As the miraculin-containing material to be extracted, freeze-dried miracle fruit sarcocarp is preferably employed. However, fresh miracle fruit sarcocarp can also be used therefor.

The extract obtained by the above-mentioned procedure, which contains NaCl, is then desalted by, for example, dialysis, ultrafiltration (including reverse osmosis, ion exchange membrane electrodialysis, etc.) or gel filtration. After freeze-drying, a crude, white-colored miraculin powder which is scarcely colored can be obtained. When sealed with a moisture absorbent such as silica gel or enclosed together with, for example, nitrogen gas so as to prevent water absorption, this powder can stably maintain its sweetness-inducing activity for 3 months or longer and be fully usable in practice. The purity of the desalted miraculin extract can be further elevated by various precipitation methods such as salting out with ammonium sulfate, sodium sulfate or potassium phosphate, isoelectric precipitation and coprecipitation together with water soluble polymers such as polyethyleneglycol or dextran. Alternatively, it may be further purified by various chromatographic procedures such as ion exchange chromatography, affinity chromatography, gel filtration chromatography, hydrophobic chromatography, hydrogen bond chromatography and chromato-focusing chromatography, membrane separation procedures based on electrophoresis or molecular weight fractionation, or by using membrane reactors to which an affinity carrier or an ion exchange carrier is bound.

The miraculin extract obtained by the process according to the present invention is scarcely colored. By using the various purification procedures as cited above, therefore, miraculin of a purity of 99% or above can be easily obtained therefrom.

The coloration of the miraculin extract or the miraculin powder obtained therefrom is caused by the impurities contained in miracle fruit sarcocarp which are liable to form rigid complexes together with the miraculin. After forming the complexes, they turn into pale brown- or dark brown-colored impurities and result in a decrease in the sweetness-inducing activity of miraculin. From the complexes of impurities with miraculin once formed, the impurities can be hardly eliminated. Although it is considered that the impurities which are liable to form complexes with miraculin include flavonoid dyes, acidic substances and complexes thereof, these substances have never been identified so far. In addition, various acidic substances capable of excessively lowering the pH value also result in a decrease in the sweetness-inducing activity of miraculin.

However, the problems caused by the impurities which are liable to form complexes with miraculin and the effects of the various acidic substances can be easily solved by the present invention wherein miraculin is extracted with an acidic buffer salt aqueous solution without washing with water as a pre-extraction treatment.

Namely, the dissociation of the reactive groups in these impurities, which are liable to form complexes with miraculin and thus cause coloration, is inhibited or suppressed under acidic conditions with the use of an acidic buffer salt aqueous solution. Thus the activities of these impurities are remarkably lowered or inactivated and, as a result, their ability to bind miraculin is suppressed. Consequently, a highly transparent miraculin extract can be obtained. On the other hand, the problem of the decrease in the pH value of the extract, which exerts undesirable effects on the miraculin activity, can be solved thereby, since the aqueous buffer solution prevents the excessive decrease in pH due to the various acidic substances contained in the sarcocarp and thus the decrease in the activity of the miraculin component contained in the extract can be also prevented. According to the present invention, the effects of the impurities which are liable to form complexes together with miraculin can be suppressed and the extraction pH can be maintained at a preferable level. Thus a miraculin powder of excellent properties which is scarcely colored and has a stable sweetness-inducing activity can be easily obtained without washing with water.

### BEST MODE FOR CARRYING OUT THE INVENTION

To further illustrate the present invention in greater detail, the following Examples will be given. However, it is to be understood that the present invention is not restricted thereto so long as it departs from the spirit thereof. For comparison, those having a pH value excluded from the range as specified in the present invention were also evaluated.

### EXAMPLE 1

Buffer salt aqueous solutions of various pH values as listed in Table 1 (each containing 0.5 M of NaCl) were added at a temperature of about 0°C (in ice water) to freeze-dried sarcocarp of miracle fruit (*Richadella dulcifica*) at a ratio of 15 ml per gram of the freeze-dried sarcocarp. After repeatedly homogenizing with an ultrasonic wave (20 kHz) for 30 seconds 4 times, the homogenized mixture was extracted by centrifuging at 10,000 rpm for 45 minutes. After the completion of the centrifugation, the supernatant was collected and referred to as the extract. Further, the residue was extracted by the same procedure and the supernatant was collected. These two supernatants extracted with the buffer salt aqueous solution of each pH value were combined and referred to as the miraculin extract.

**Table 1**

| Preparation Of Aqueous Buffer Salt Solutions Of Various PH Values | | | |
|---|---|---|---|
| No. | pH | Conc. (M) | Buffer Solution (containing 0.5 M of NaCl) |
| 1 (Comp.) | 3.1 | 0.1 | citric acid/monosodium citrate |
| 2 (Ex.) | 3.5 | 0.1 | monopotassium phthalate (adjusted with 1 N HCl) |
| 3 (Ex.) | 4.5 | 0.1 | acetic acid/sodium acetate |
| 4 (Ex.) | 5.1 | 0.1 | acetic acid/sodium acetate |
| 5 (Ex.) | 5.5 | 0.1 | monosodium citrate/disodium citrate |
| 6 (Comp.) | 6.5 | 0.1 | sodium hydrogencarbonate (adjusted with 1 N HCl) |
| 7 (Comp.) | 7.5 | 0.1 | monosodium phosphate/disodium phosphate |
| 8 (Comp.) | 8.3 | 0.1 | glycylglycine (adjusted with 1 N NaOH) |
| 9 (Comp.) | 9.2 | 0.1 | boric acid/sodium borate |
| 10 (Comp.) | 10.1 | 0.1 | monosodium carbonate/disodium carbonate |

Now, the methods for preparing these aqueous buffer salt solutions of various pH values will be described. Each reagent used here was a first-grade product of Wako Pure Chemicals, Inc.

### (1) Preparation of aqueous buffer salt solution of pH 3.1

A 0.1 M citric acid aqueous solution and a 0.1 M monosodium citrate aqueous solution were mixed together while monitoring the pH value of the mixture with a pH meter until the pH value reached 3.1. Then NaCl was added to the mixture in such a manner as to give an NaCl concentration of the solution of 0.5 M.

### (2) Preparation of aqueous buffer salt solution of pH 3.5

To 0.1 M monosodium phthalate, was added a 1 N HCl aqueous solution while monitoring the pH value of the mixture with a pH meter until the pH value reached 3.5. Then NaCl was added to the mixture in such a manner as to give an NaCl concentration of the solution of 0.5 M.

### (3) Preparation of aqueous buffer salt solution of pH 4.5

A 0.1 M acetic acid aqueous solution and a 0.1 M sodium acetate aqueous solution were mixed together while monitoring the pH value of the mixture with a pH meter until the pH value reached 4.5. Then NaCl was added to the mixture in such a manner as to give an NaCl concentration of the solution of 0.5 M.

### (4) Preparation of aqueous buffer salt solution of pH 5.1

A 0.1 M acetic acid aqueous solution and a 0.1 M sodium acetate aqueous solution were mixed together while monitoring the pH value of the mixture with a pH meter until the pH value reached 5.1. Then NaCl was added to the mixture in such a manner as to give an NaCl concentration of the solution of 0.5 M.

### (5) Preparation of aqueous buffer salt solution of pH 5.5

A 0.1 M monosodium citrate aqueous solution and a 0.1 M disodium citrate aqueous solution were mixed together while monitoring the pH value of the mixture with a pH meter until the pH value reached 5.5. Then NaCl was added to the mixture in such a manner as to give an NaCl concentration of the solution of 0.5 M.

### (6) Preparation of aqueous buffer salt solution of pH 6.5

To a 0.1 M sodium hydrogencarbonate aqueous solution, was added a 1 N HCl aqueous solution while monitoring the pH value of the mixture with a pH meter until the pH value reached 6.5. Then NaCl was added to the mixture in such a manner as to give an NaCl concentration of the solution of 0.5 M.

### (7) Preparation of aqueous buffer salt solution of pH 7.5

A 0.1 M monosodium phosphate aqueous solution and a 0.1 M disodium phosphate aqueous solution were mixed together while monitoring the pH value of the mixture with a pH meter until the pH value reached 7.5. Then NaCl was added to the mixture in such a manner as to give an NaCl concentration of the solution of 0.5 M.

### (8) Preparation of aqueous buffer salt solution of pH 8.3

To a 0.1 M glycylglycine aqueous solution (prepared by dissolving 13.2 g of glycylglycine in 1,000 ml of pure water), was added a 1 N NaOH aqueous solution while monitoring the pH value of the mixture with a pH meter until the pH value reached 8.3. Then NaCl was added to the mixture in such a manner as to give an NaCl concentration of the solution of 0.5 M.

### (9) Preparation of aqueous buffer salt solution of pH 9.2

A 0.1 M boric acid aqueous solution and a 0.1 M sodium borate aqueous solution were mixed together while monitoring the pH value of the mixture with a pH meter until the pH value reached 9.2. Then NaCl was added to the mixture in such a manner as to give an NaCl concentration of the solution of 0.5 M.

### (10) Preparation of aqueous buffer salt solution of pH 10.1

A 0.1 M monosodium carbonate aqueous solution and a 0.1 M disodium carbonate aqueous solution were mixed together while monitoring the pH value of the mixture with a pH meter until the pH value reached 10.1. Then NaCl was added to the mixture in such a manner as to give an NaCl concentration of the solution of 0.5 M.

A 5 ml amount of a miraculin extract obtained by using the buffer salt aqueous solution of each pH value thus obtained was desalted by desalting gel filtration chromatography (a product of Pharmacia Co.) equilibrated with pure water to thereby give 7 ml of a desalted solution which was referred to as the desalted miraculin extract.

Regarding the desalted miraculin extract of each pH value, the degree of coloration was measured by using a chromaticity diagram specified in JIS Z 8721, and the sweetness-inducing activity was evaluated by the procedure as given below. Table 2 shows the obtained results, wherein the coloration "-" means "colorless and transparent".

As Table 2 shows, among the buffer salt aqueous solutions of various pH values, the acidic buffer salt aqueous solutions according to the present invention caused no coloration and gave high sweetness-inducing activities.

### Procedure For Coloration Test

A 3.5 ml amount of a desalted miraculin extract was introduced into a test tube (19 mm in inner diameter, 21 mm in outer diameter) and the test tube was placed vertically under a natural light while avoiding the direct rays of the sun. Then a JIS Z 8721 chromaticity diagram indicating the hue, lightness and chromaticness was placed adjacent to the tube and the degree of coloration of the miraculin extract was evaluated from a sideward direction.

### Procedures For Sweetness-Inducing Activity Evaluation

(1) 2 to 3 ml of a 0.1 M citric acid solution was kept in the mouth and the sour taste was confirmed.
(2) The citric acid solution was ejected and the mouth was rinsed with water.
(3) 2.5 ml of the desalted miraculin extract was kept in the mouth.
(4) The miraculin extract was rolled on the tongue for 1 minute so as to allow the tongue to fully adsorb it.
(5) The miraculin extract was ejected and the mouth was rinsed with water.
(6) 2 to 3 ml of a 0.1 M citric acid solution was kept in the mouth and the sweetness thus sensed was remembered.
(7) 2 to 3 ml of each of standard sweet solutions (0.1, 0.2, 0.3, 0.4 and 0.5 M solutions of sucrose) was kept in the mouth and the one showing sweetness comparable to that sensed when the above-mentioned 0.1 M citric acid solution was kept in the mouth was selected. Then, the sucrose concentration of the selected standard solution was referred to as the sweetness-inducing activity.

**Table 2**

| Degrees Of Coloration Of Desalted Extracts | | | |
|---|---|---|---|
| No. | pH | Coloration | Sweetness-Inducing Activity |
| 1 (Comp.) | 3.1 | - | 0.2 |
| 2 (Ex.) | 3.5 | - | 0.3 |
| 3 (Ex.) | 4.5 | - | 0.3 |
| 4 (Ex.) | 5.1 | - | 0.3 |
| 5 (Ex.) | 5.5 | - | 0.3 |
| 6 (Comp.) | 6.5 | 5YR 8/2 | 0.2 |
| 7 (Comp.) | 7.5 | 5Y 8/3 | 0.1 |
| 8 (Comp.) | 8.3 | 2.5Y 7/6 | 0.1 |
| 9 (Comp.) | 9.2 | 2.5Y 7/8 | 0.1 |
| 10 (Comp.) | 10.1 | 10YR 6/8 | 0.1 |

### Standards For Evaluation Of Coloration (JIS Z 8721):

For example, in (10YR 6/8), 10YR indicates "hue", 6 indicates "lightness", and 8 indicates "chromaticness".
Hue: 4 grades (2.5, 5, 7.5, 10)
Y means "yellow" while R means "red".
Lightness: eight grades (2, 3, 4, 5, 6, 7, 8, 9), which means "Dark ↔ Light" in this order.
Chromaticness: When the hue is 5 or 10, it is represented by ten grades (N, 1, 2, 3, 4, 6, 8, 10, 12, 14), and when the hue is 2.5 or 7.5, it is represented by nine grades (N, 2, 3, 4, 6, 8, 10, 12, 14), which means "Light (pale) ↔ Dark (deep)" in this order.

Next, a 3.5 ml amount of each of the desalted miraculin extracts of various pH values was transferred into a 5 ml vial and freeze-dried therein. The degree of coloration of the crude miraculin powder thus obtained was determined by placing a JIS Z 8721 chromaticity diagram adjacent to the vial containing the crude miraculin powder. Table 3 summarizes the results.

In a case of using the acidic buffer salt aqueous solutions according to the present invention, a white crude miraculin powder which was not colored, was obtained.

**Table 3**

| Degrees Of Coloration Of Freeze-Dried Crude Powders Obtained From Desalted Miraculin Extracts | | |
|---|---|---|
| No. | pH | Coloration |
| 1 (Comp.) | 3.1 | 2.5R 9/N |
| 2 (Ex.) | 3.5 | 2.5R 9/N |
| 3 (Ex.) | 4.5 | 2.5R 9/N |
| 4 (Ex.) | 5.1 | 2.5R 9/N |
| 5 (Ex.) | 5.5 | 2.5R 9/N |
| 6 (Comp.) | 6.5 | 2.5Y 7/4 |
| 7 (Comp.) | 7.5 | 5YR 7/3 |
| 8 (Comp.) | 8.3 | 7.5Y 7/4 |
| 9 (Comp.) | 9.2 | 7.5Y 7/4 |
| 10 (Comp.) | 10.1 | 5Y 5/4 |

The miraculin content of each of the miraculin extracts of various pH values was determined by the sandwich method [P.K. Nakane and A. Kawaoi, (1974) Peroxidase-Labeled antibody: a new method of conjugation, J. Histochem. Cytochem., 22, 1084 - 1091], wherein anti-miraculin IgG (antibody: immunoglobulin) was bound to an enzyme (POD: peroxidase) and thus labeled. The results were compared with each other. Table 4 summarizes the results.

The extracts obtained by using the acidic buffer salt aqueous solutions according to the present invention showed larger miraculin contents than those obtained by using other acidic buffer salt aqueous solutions. These facts clearly indicate that miraculin contaminated with less impurities can be stably extracted by using the acidic buffer salt aqueous solutions according to the present invention.

**Table 4**

| Miraculin Contents Of Extracts | | |
|---|---|---|
| No. | pH | Miraculin Content (µg/ml) |
| 1 (Comp.) | 3.1 | 160 |
| 2 (Ex.) | 3.5 | 257 |
| 3 (Ex.) | 4.5 | 203 |
| 4 (Ex.) | 5.1 | 202 |
| 5 (Ex.) | 5.5 | 242 |
| 6 (Comp.) | 6.5 | 173 |
| 7 (Comp.) | 7.5 | 150 |
| 8 (Comp.) | 8.3 | 152 |
| 9 (Comp.) | 9.2 | 156 |
| 10 (Comp.) | 10.1 | 74 |

The miraculin contents were determined by the following method.

### Procedure For The Determination Of Miraculin Content

100 µl/well of anti-miraculin IgG was added to a 96-well microplate and allowed to stand at 4°C overnight for binding the anti-miraculin IgG into the inside wall of each well. Then, the bound anti-miraculin IgG was washed with a 0.18% aqueous solution of potassium phosphate (pH 7.5). After removing the residual solution in the well, 150 µl/well of a 1% BSA (bovine serum albumin: a product of CALBIOCHEM Co.) dissolved in a 0.18% potassium phosphate aqueous solution (pH 7.5) was added and allowed to stand at room temperature for 2 hours. After removing the 1% BSA solution in the well, 100 µl portions of miraculin solutions of known concentrations and the miraculin extracts of various pH values obtained above, each diluted 10,000-fold, were added to the anti-miraculin IgG bound to the inside of the well and allowed to stand at room temperature for 2 hours. Then the anti-miraculin IgG was washed again with a 0.18% aqueous solution of potassium phosphate (pH 7.5) to remove the residual solutions in the well. 100 µl of the labeled IgG was added thereto, followed by allowing to stand at room temperature for 2 hours. After washing with a 0.18% aqueous solution of potassium phosphate (pH 7.5) to remove the residual solution in the well, a color-development solution from a color development kit for peroxidase (a product of Sumitomo Bakelite Co., Ltd.) was added to each extract to thereby induce the color development. After 3 minutes, a stopping solution from the same kit was added to thereby cease the reaction. Then, the absorbance at 405 nm was measured with an immunoreader. The miraculin content of each extract was then determined based on a calibration curve formed by using the data of the above-mentioned miraculin solutions of known concentrations (0.5, 1.0, 2.0, 5.0, 10, 20, 50, 100, 200, 500 ng/ml).

Now, methods for preparing the anti-miraculin IgG and the labeled IgG employed above will be described.

### Preparation Of Anti-Miraculin IgG

A 250 µg amount of purified miraculin of 99.9% or greater in purity was emulsified with the same amount of complete Freund's adjuvant and injected into a rabbit (body weight: 3 - 3.5 kg). After 10 days and 20 days, 250 µg portions of incomplete Freund's adjuvant were further injected into the rabbit. One week after the final injection, the blood of the animal was collected and thus anti-miraculin serum was obtained.

A 7 ml amount of this serum was added to 5 ml of protein A (a product of Seikagaku Kogyo K.K.) equilibrated with 0.05 M tris hydrochloride (pH 8.5, 0.15 M NaCl). Then the mixture was slowly stirred with a stirrer for 2 to 3 hours in a cold room (4 to 10°C) so as to allow the protein A to adsorb the anti-miraculin IgG. The protein A adsorbing the anti-miraculin IgG was packed in a column and IgG was eluted with 0.05 M sodium acetate (pH 4.0, 0.15 M NaCl). Thus, approximately 25 mg of purified anti-miraculin IgG was obtained.

### Preparation Of Labeled IgG

A 4 mg amount of POD (peroxidase, a product of Boehringer Co.) was dissolved in 1 ml of pure water. A 0.2 ml amount of 0.1 M sodium periodate was added thereto. After allowing to stand at room temperature for 20 minutes and inserting in a dialysis tube, the mixture was dialyzed against a 1 mM sodium acetate buffer solution (pH 4.4) overnight.

After the completion of the dialysis, to bind the POD to the anti-miraculin IgG, a 0.2 M sodium carbonate buffer solution (pH 9.5) was added to the POD and thus the pH value was adjusted to about 9. To the POD, 1 ml of a 0.01 M sodium carbonate buffer solution (pH 9.5) containing 8 mg of IgG was added and the mixture was shaken at room temperature for 2 hours. To separate the anti-miraculin IgG bound to the POD, 0.1 ml of sodium boron hydride (4 mg) dissolved in 1 ml of pure water was added. After allowing to stand at 4°C for 2 hours, the mixture was added to gel filtration chromatography (Sephacryl s-200 column, equilibrated with a 10 mM sodium phosphate buffer solution of pH 6.8) and the eluate was collected in 2 ml portions. The absorbances of each fraction at 280 nm (absorption due to protein), 403 nm (absorption due to POD, more accurately, iron in POD) and 405 nm (absorption due to POD activity) were measured. In fractions showing these three peaks, IgG was bound to active POD and thus the labeled IgG was contained therein. The fractions showing these three peaks were combined together. In order to stabilize the labeled IgG, BSA (powdered bovine serum albumin for immunoassay, a product of Wako Pure Chemicals, Inc.) was further added thereto so as to give a final BSA concentration of 1 mg/ml. Thus labeled IgG was obtained.

### INDUSTRIAL APPLICABILITY

The present invention makes it possible, by effecting extraction with an acidic buffer salt aqueous solution, to suppress the reactivity of impurities which are liable to form complexes together with miraculin and thus cause coloration or a decrease in the sweetness-inducing activity without causing any excessive decrease in pH due to various acidic substances contained in miracle fruit. Thus a decrease in the miraculin component activity can be prevented thereby. Further, the present invention further makes it unnecessary to remove the impurities as a pre-extraction treatment. Compared with conventional methods, scarcely colored miraculin having a stable sweetness-inducing activity can be efficiently produced thereby. Thus, the present invention largely contributes to the effective production of miraculin on an industrial scale.

According to the present invention, miraculin which is scarcely colored and has a stable sweetness-inducing activity can be obtained. Thus purified miraculin of a high purity can be easily produced industrially. Therefore, the present invention advantageously contributes to the production of foods, drinks and medicines.

## Claims

1. A process for producing miraculin which comprises extracting miraculin from a miraculin-containing material with an acidic buffer salt aqueous solution.

2. A process for producing miraculin as claimed in Claim 1, wherein the pH value of said acidic buffer salt aqueous solution ranges from 3.5 to 5.5.
